# FASCICULE DE BREVET EUROPEEN

(11) **EP 2 344 248 B1**
(45) Date de publication et mention de la délivrance du brevet: **24.07.2013**
(21) Numéro de dépôt: 09741394.2
(22) Date de dépôt: 10.09.2009
(51) Int. Cl.: A61N 5/06, A61B 18/18, A61B 18/20, A61B 18/22, A61B 19/00

(54) **PIÈCE A MAIN A BLOC OPTIQUE D'APPAREIL DE PHOTOTRAITEMENT DE LA PEAU**
HANDSTÜCK MIT OPTIKEINHEIT EINER VORRICHTUNG FÜR DIE PHOTOTHERAPIE DER HAUT
HANDPIECE WITH OPTICAL UNIT OF A SKIN PHOTOTREATMENT DEVICE

(30) Priorité: 12.09.2008 FR 0856152; 09.09.2009 FR 0956124
(43) Date de publication de la demande: 20.07.2011
(73) Titulaire: Dermeo, 75018 Paris (FR)
(72) Inventeur: HOTTINGER, Christophe, F-75009 Paris (FR); TANNOUS, Pascale, F-75009 Paris (FR)
(74) Mandataire: Cabinet HERRBURGER
(86) Numéro de dépôt international: PCT/FR2009/051700
(87) Numéro de publication internationale: WO 2010/029258

(56) Documents cités:
- EP-A- 1 535 582
- WO-A-03/043514
- WO-A-2007/007167
- WO-A-2008/012519
- WO-A-2008/070747
- WO-A-2008/088792
- WO-A-2008/088795
- US-A1- 2007 198 004

## Description

### Domaine de l'invention

La présente invention concerne une pièce à main d'appareil de phototraitement de la peau, pièce formée d'une coque avec une poignée reliée à un faisceau de câbles d'alimentation électrique et de tubes de fluide caloporteur et une tête, recevant une cartouche munie de la source lumineuse commandée et d'un guide de lumière alimenté par la source lumineuse et dont la surface de sortie constitue la surface d'émission du signal lumineux vers la surface de la peau à traiter.

### Etat de la technique

On connaît déjà différentes réalisations de tels appareils de phototraitement de la peau pour effectuer des traitements cosmétiques, médicaux ou thérapeutiques. Ces appareils comportent en général une partie constituant un poste fixe (bien qu'installé le cas échéant sur des roulettes) reliée à une pièce à main qui constitue l'élément utilisé pour effectuer le traitement. Cette pièce à main est rapprochée de la surface de la peau à traiter ou est placée sur celle-ci pour lui appliquer des signaux lumineux. Ces signaux lumineux peuvent avoir des caractéristiques physiques très différentes selon leur longueur d'ondes, l'intensité du faisceau, la fréquence impulsionnelle du faisceau et autres paramètres.

La lumière est émise par une lampe flash logée dans la pièce à main. Cette lampe est alimentée de façon commandée à partir d'une source se trouvant dans le poste fixe de l'appareil. Un circuit de refroidissement à liquide est également prévu. Ce circuit passe autour de la lampe source de lumière et dans un échangeur de chaleur dans le poste fixe, avec une mise en circulation du fluide par une pompe.

Il existe selon le document WO2007/007167, une cartouche de lampe flash qui comporte dans une version (figure 3), un bloc optique solidaire de la cartouche.

Selon le document EP 1 535 582, on connaît une pièce à main qui se compose pratiquement de deux parties, l'enveloppe de la pièce à main et la partie électronique et optique constituée par un module que l'on glisse dans la pièce à main et que l'on fixe ainsi.

Le remplacement de la cartouche nécessite l'enlèvement de cette pièce globale puis le démontage de la cartouche, le remontage d'une cartouche neuve et enfin la remise en place de ce boîtier dans l'ouverture de la pièce à main. Cette solution connue a l'inconvénient d'être relativement complexe et seul le spécialiste pourra enlever la partie amovible de la pièce à main puis l'ouvrir pour en extraire la cartouche et la remplacer le cas échéant.

Pour changer d'optique, il faut démonter toute la cartouche, mettre en place la nouvelle optique puis réintégrer l'ensemble dans la poignée de la pièce à main.

### But de l'invention

La présente invention a pour but de développer une pièce à main pour un appareil de phototraitement de la peau permettant d'adapter de manière simple la pièce à main à différents traitements à effectuer pour concentrer, filtrer ou émettre un faisceau lumineux le plus approprié au traitement sans nécessiter de transformations importantes de la pièce à main et surtout permettre à un opérateur d'effectuer ces modifications de la pièce à main d'une manière particulièrement simple.

### Exposé et avantages de l'invention

A cet effet, la présente invention concerne une pièce à main du type défini ci-dessus caractérisée en ce qu'elle comprend
- un logement débouchant réalisé dans la coque, arrivant sous la surface d'émission de la cartouche et muni de moyens de guidage et d'organes d'enclipsage,
- un bloc optique interchangeable portant le guide de lumière et engagé de façon amovible dans le logement, ce bloc optique ayant un boîtier recevant le guide de lumière et muni de surfaces de guidage et d'enclipsage pour coopérer avec les moyens de guidage et les organes d'enclipsage escamotable du logement.

Cette pièce à main avec un bloc optique amovible permet d'effectuer très rapidement une transformation de la pièce à main en fonction du traitement à effectuer. Pour cette transformation, il suffit d'extraire le bloc optique pour le remplacer par un le bloc optique dont les caractéristiques optiques correspondent au traitement à effectuer. Cela permet d'alterner dans de bonnes conditions et sans perte de temps, des traitements différents. Aucune intervention n'est nécessaire sur la pièce à main même ; il n'est pas nécessaire de l'ouvrir, de brancher, ou de débrancher la pièce à main. L'appareil est simplement au repos pour que la source lumineuse ne soit pas alimentée.

Cette sécurité peut en outre être obtenue à l'aide d'un capteur de présence qui détecte l'engagement d'un bloc optique dans le logement de la pièce à main. En l'absence de bloc optique, le capteur transmet un signal à l'appareil qui bloque ainsi le fonctionnement de la source lumineuse pour éviter un éventuel éblouissement ou accident oculaire.

En fin de travail, il est également possible d'extraire le bloc optique pour le ranger de façon à le protéger et de le remplacer par un bloc optique aveugle, c'est-à-dire un couvercle ayant la forme du boîtier du bloc optique pour se placer dans le logement de la pièce à main, fermer celui-ci et éviter la pénétration de poussières ou, le cas échéant, que le cartouche ne soit accidentellement endommagé.

Le boîtier de ce couvercle peut comporter avantageusement un dégagement à l'emplacement du détecteur de présence du bloc optique pour que, bien qu'il soit mis en place dans le logement du bloc optique, ce boîtier ne libère pas le fonctionnement de la source lumineuse.

Suivant une autre caractéristique avantageuse, les organes d'enclipsage sont constitués par des butées à ressort en forme de piston dont la tête est en saillie de la surface de guidage du logement et le boîtier du bloc optique comporte des surfaces de guidage munies d'une cavité formant une rampe pour recevoir la tête du piston de l'organe d'enclipsage associé.

Ces moyens sont particulièrement simples et fiables à utiliser. Ils ne nécessitent pas d'efforts particuliers pour l'extraction ou la mise en place du bloc optique grâce aux surfaces formant des rampes coopérant avec les organes d'enclipsage. Ces moyens sont très fiables et permettent un grand nombre de cycles de fonctionnement.

Suivant une autre caractéristique avantageuse, le boîtier est constitué par un élément de forme parallélépipédique, de section globalement rectangulaire, composé d'une première partie avec un fond et deux côtés et d'une seconde partie en forme de couvercle pour constituer entre elles une cavité débouchant des deux côtés pour recevoir le guide de lumière, les deux parties étant fixées l'une à l'autre pour bloquer le guide de lumière et les côtés formant les surfaces de guidage et d'enclipsage.

Cette forme de réalisation du boîtier permet de recevoir différentes formes de guide de lumière sans que la forme du boîtier ne doive être préparée pour s'adapter à la mise en place amovible du bloc optique dans la coque de la pièce à main.

Suivant une autre caractéristique avantageuse, le boîtier comporte des rainures réalisées dans les grands côtés pour coopérer avec des nervures de guidage homologues du logement de la coque.

Suivant une autre caractéristique avantageuse, dans sa surface qui dépasse du logement de la coque lorsque le bloc optique y est en place, le boîtier comporte sur deux grandes faces, des cavités de préhension.

Le bloc optique amovible selon l'invention comporte de préférence un guide de lumière en verre quartz ou verre saphir. Il peut assurer ou non une filtration optique pour échantillonner le spectre lumineux en fonction des applications souhaitées.

Suivant une autre caractéristique, le bloc optique est muni d'un système de refroidissement du guide de lumière utilisant par exemple l'effet Peltier.

Suivant une caractéristique avantageuse, le bloc optique comporte une plaquette-interface appliquée contre la face d'entrée du guide de lumière et ayant des caractéristiques optiques pour la transmission de la lumière entre la source lumineuse de la cartouche et le guide de lumière.

Cette plaquette-interface permet de compléter le guide de lumière du bloc optique et d'assurer les caractéristiques optiques à la combinaison qu'elle forme avec le guide de lumière de manière à pouvoir utiliser un même guide de lumière pour obtenir différentes caractéristiques optiques grâce à différentes plaquettes-interfaces. Ces plaquettes-interfaces peuvent avoir une face traitée ou être teintées dans la masse pour constituer un filtre optique correspondant à une certaine longueur d'onde. La lumière filtrée sera plus ou moins précise selon que l'on utilise une plaquette munie d'un revêtement filtrant ou d'une plaquette teintée dans la masse.

La plaquette munie du revêtement filtrant est avantageusement tournée avec sa face revêtue contre le guide de lumière de sorte que le revêtement ne risque pas d'être touché et de subir une attaque chimique sous l'effet du contact avec un doigt de l'opérateur.

De plus, la réalisation du guide de lumière sous la forme d'un élément optiquement neutre sans effet de filtrage, facilite considérablement la réalisation du bloc optique ainsi que la gestion des stocks puisque pour les différentes caractéristiques filtrantes, il suffira de modifier la plaquette-interface. Cette modification est d'autant plus simple que la plaquette-interface est glissée dans les deux rainures du boîtier du bloc optique.

La plaquette-interface assure également une protection mécanique de la face d'entrée de lumière du guide de lumière contre lequel elle s'applique.

Enfin, la plaquette a une fonction mécanique de blocage du guide de lumière dans le boîtier puisque le guide de lumière ne pourra s'échapper du boîtier dans la direction de la plaquette-interface.

On évite ainsi d'avoir à bloquer le guide de lumière dans le boîtier par des points de colle.

On évite donc non seulement une opération supplémentaire mais aussi l'agression des deux faces du bloc optique par la colle qui modifie l'interface à ce niveau et favorise l'émission de rayons lumineux par les faces couvertes de colle du guide de lumière. Cette évasion réduit le rendement optique du guide de lumière et produit l'échauffement des faces latérales du guide de lumière et des pièces en contact avec celles-ci. En supprimant les points de colle, on supprime cette source de fuite de lumière.

Suivant une autre caractéristique avantageuse, le bloc optique a un boîtier dont la première partie comporte un logement recevant le guide optique et au-dessus du logement, deux rainures dans les petits côtés recevant la plaquette-interface.

Cette réalisation est particulièrement intéressante pour remplacer ou changer la plaquette-interface puisqu'il suffit de démonter le couvercle, en général vissé, pour pouvoir extraire la plaquette et la remplacer par une plaquette neuve ou une plaquette ayant d'autres caractéristiques optiques que la plaquette précédente.

Suivant une autre caractéristique avantageuse, le boîtier du bloc optique comporte des surfaces de guidage munies d'une cavité formant une rampe et précédées dans le sens de l'engagement du bloc optique dans son logement de la coque par deux bossages dont le premier assure le positionnement du bloc optique dans l'entrée du logement et le second bossage délimite la cavité recevant la tête du piston de chacun des organes d'enclipsage.

Cette réalisation des deux petits côtés du boîtier du bloc optique facilite l'engagement précis du bloc optique dans son logement de la pièce à main pour bien positionner le bloc optique par rapport au cartouche logeant la lampe à décharge qui génère le signal lumineux à transmettre. Grâce à cette réalisation, on évite des interfaces d'air entre la fenêtre du cartouche et la plaquette-interface ainsi qu'entre la plaquette-interface et la face d'entrée du guide de lumière.

En outre, la surface de la plaquette-interface est avantageusement plus grande que celle de la face d'entrée du guide de lumière de sorte que la plaquette dépasse largement de la face d'entrée des deux côtés de manière à bien couvrir la fenêtre du cartouche et en déborder pour que la venue en appui de la plaquette-interface contre la fenêtre se fasse toujours par un contact plan, l'opération étant favorisée par la forme des surfaces de guidage du bloc optique.

Suivant une autre caractéristique avantageuse, le bloc optique comporte sur les deux faces du guide de lumière, un réflecteur en forme de plaque métallique appliquée contre chaque grande face du guide de lumière pour protéger le boîtier contre la surchauffe.

Les plaques métalliques installées sur chaque grande face du guide de lumière constituent un moyen supplémentaire évitant la sortie de lumière par les grandes faces, sorties inévitables quelle que soit la finition en poli de miroir des faces du guide de lumière. On protège ainsi le boîtier du bloc optique contre une surchauffe locale.

### Dessins

La présente invention sera décrite ci-après de manière plus détaillée à l'aide de modes de réalisation d'une pièce à main à bloc optique amovible et interchangeable représentés schématiquement dans les dessins annexés dans lesquels :
- la figure 1 est une vue de côté d'un premier mode de réalisation de la pièce à main selon l'invention,
- la figure 2 est une vue en coupe longitudinale par le plan médian de la pièce à main de la figure 1,
- la figure 3A est une coupe selon le plan IIIA IIIA de la figure 2,
- la figure 3B est une coupe selon le plan IIIB IIIB de la figure 1,
- la figure 4 est une vue en perspective d'un bloc optique pour la pièce à main de la figure 1,
- la figure 5 est une vue éclatée du bloc optique de la figure 4,
- la figure 6 est une vue en perspective d'une variante de réalisation d'un bloc optique selon l'invention,
- la figure 7 est une vue en perspective correspondant à la figure 6 mais dont le couvercle du boîtier du bloc optique a été enlevé,
- la figure 8 est une coupe de la partie de la pièce à main et du bloc optique.

### Description d'un mode de réalisation de l'invention

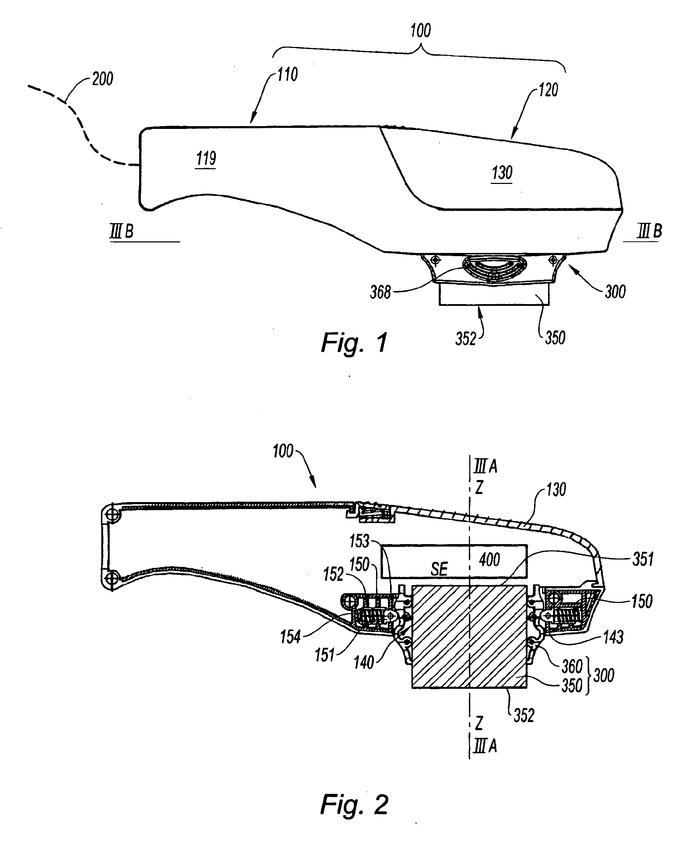

Selon la figure 1, l'invention concerne une pièce à main pour un appareil de phototraitement cosmétique, médical/thérapeutique de la peau. Cette pièce à main 100 est reliée à l'appareil proprement dit, non représenté, par un faisceau de câbles et de tuyaux 200 sortant de l'extrémité de la coque 110 ; la pièce à main forme une poignée 110, et une tête 120 logeant une cartouche avec une source lumineuse commandée, non représentée, qui fournit les signaux lumineux de traitement. Cette source lumineuse est en contact avec un bloc optique 300 amovible, et interchangeable, portant un guide de lumière 350, par exemple de forme prismatique, dont la face 351 (figure 2) à l'intérieur de la coque 110 constitue l'entrée des signaux lumineux émis par la source lumineuse et dont la face 352 apparaissant à l'extérieur constitue la face d'émission des signaux lumineux ; cette face est dirigée vers la surface de peau à traiter.

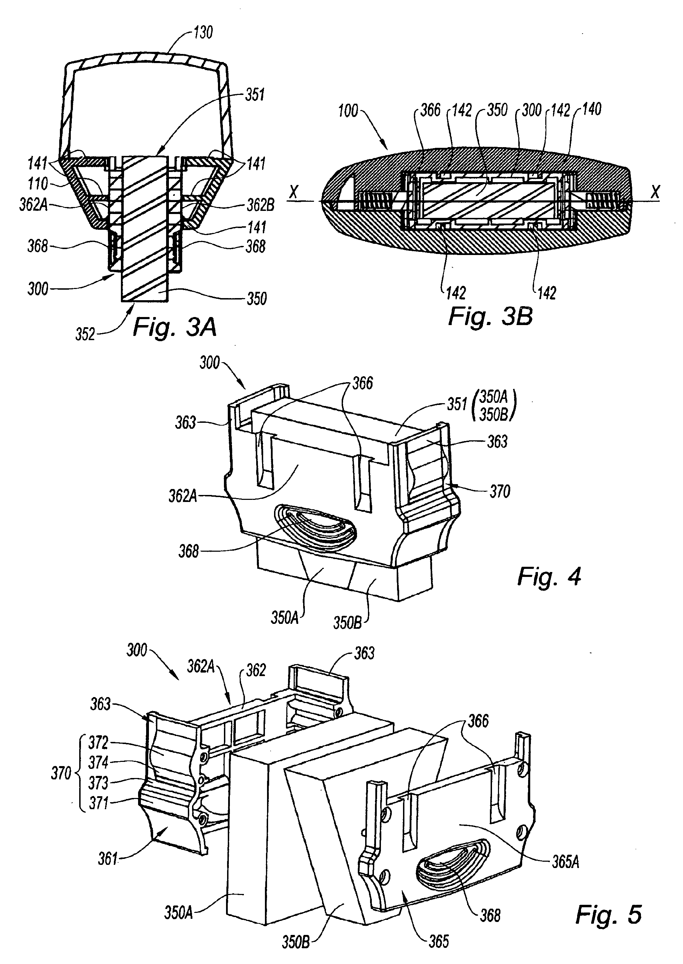

Les vues en coupe des figures 2 et 3 montrent la structure de la pièce à main 100 pour les parties limitées à celles concernant la présente invention.

La coque 110 est constituée par l'assemblage de parties 110a, b moulées séparément en matière plastique et d'un capot amovible 130 pour accéder à la cartouche 400.

Selon les figures 2, 3A, 3B, sous la surface d'émission SE de la cartouche, la coque 110 a un logement 140 débouchant à l'extérieur de la coque et recevant le bloc optique 300 ; le bloc optique 300 a en vue de dessus (section horizontale), une forme sensiblement rectangulaire (figure 3B) dont la grande longueur est dirigée dans l'axe longitudinal XX (dit axe horizontal) de la pièce à main. Le logement 140 est délimité sur ses grands côtés, par des nervures 141 horizontales (figure 3A) et des guides 142 (figure 3B) orientés dans l'axe ZZ (dit axe vertical) du logement, perpendiculaire à l'axe XX ; les petits côtés ont des surfaces de guidage 143 et au moins un et, de préférence, deux éléments d'enclipsage 150.

Les nervures 142 (figure 3B) viennent en saillie dans le logement 140 pour coopérer avec des rainures 366 du boîtier 360 du bloc optique 300. Les surfaces de guidage 143 sur les petits côtés sont deux surfaces planes, également parallèles à l'axe ZZ et dont dépasse la tête arrondie 153 du piston 151 de chaque élément d'enclipsage 150. Les pistons 151 coulissent dans des logements transversaux 152 (direction XX de la coque 110) et leur tête arrondie 153, le cas échéant constituée par une bille, est poussée par un ressort 154 pour dépasser de la surface de guidage 143 ou s'escamoter dans le logement 152 pour permettre le passage des parties en relief des petits côtés 363 du boîtier 360 du bloc optique 300.

Le bloc optique 300 (figures 4 et 5) est constitué par le boîtier 360 recevant le guide de lumière 350. Le guide de lumière 350 est une pièce en un matériau transparent ayant les caractéristiques optiques souhaitées, par exemple en quartz. Le guide de lumière 350 a une forme parallélépipédique ou prismatique avec une surface d'entrée 351 et une surface de sortie 352 du signal lumineux. La surface d'entrée 351 est destinée à venir contre la fenêtre du cartouche 400 logeant la source lumineuse et la surface de sortie 352 est celle par laquelle le signal lumineux est dirigé vers la surface de la peau à traiter.

Le boîtier 360 est formé d'une première partie 361 composée d'un fond rectangulaire 362 bordé de deux petits côtés opposés 363, (les deux autres côtés du haut et du bas étant ouverts), et d'une seconde partie 365, en forme de couvercle fixé aux cotés 363 de la première partie en délimitant le logement ouvert dans lequel se place et se bloque le guide de lumière 350. Le guide de lumière 350 a une forme parallélépipédique (350A) ou prismatique (350B) ou tout autre forme nécessaire au phototraitement. Les guides de lumière 350 diffèrent par leurs caractéristiques géométriques et optiques. De préférence, ces guides de lumière ont une forme extérieure permettant leur mise en place dans le même type de boîtier 360 pour simplifier la fabrication. Les guides de lumière 350 sont installés solidairement dans le boîtier 360 pour constituer un bloc optique 300 amovible de la pièce à main pour être interchangeable en fonction de l'application.

Les deux grandes faces extérieures 362A, 365A du boîtier 360 sont identiques. Elles comportent chacune deux rainures de guidage longitudinal 366 selon la direction ZZ dans leur zone supérieure pour coopérer avec les nervures 142 du logement 140 ; dans leur partie inférieure, dépassant de la coque 110 lorsque le bloc optique 300 est mis en place, elles comportent une cavité de préhension 368 munie de nervures ou d'éléments anti-glissement, pour permettre d'extraire le bloc optique 300 de son logement 140 de la coque 110, sans toucher le guide de lumière 350 et notamment ses deux surfaces d'entrée et de sortie 351, 352. Les cavités de préhension 368 constituent des moyens de préhension s'utilisant naturellement ; elles permettent l'extraction et l'insertion d'un bloc optique 300 en l'enclipsant dans son logement avec une seule main.

La face intérieure 362B du grand côté 362 et celle 365B du couvercle 365 sont nervurées pour être rigides et tenir le guide de lumière 350.

Les petits côtés 363 du boîtier 360 sont munis de surfaces 370 définissant une cavité d'enclipsage avec une rampe pour coopérer avec les deux têtes de piston 153 qui se font face dans le logement 140.

De façon plus précise, les deux surfaces 370, opposées, se composent chacune de deux bossages 371, 372 séparés par une cuvette 373 de façon à former une rampe 374 inclinée pour que la tête de piston 153 de chaque côté assure non seulement l'enclipsage du bloc optique 300 dans le logement 140 mais s'appuie aussi contre la rampe 374 tournée vers le haut, donnant une composante de poussée vers le haut dans la direction de l'axe ZZ, pour bien appliquer le bloc optique 300 contre la surface de butée supérieure constituée par la fenêtre du cartouche 400 de la source lumineuse.

Il est également possible de réaliser un « bloc optique aveugle » formant couvercle mais sans guide de lumière que l'on engage dans le logement 140 après avoir extrait le bloc optique équipé de son guide optique. Ainsi, on ferme le logement 140 tout en évitant que la surface du guide de lumière ne risque d'abîmer dans les déplacements, travaux d'entretien effectués dans les locaux ou autres situations de ce genre. Le bloc optique peut être rangé avec les autres blocs optiques destinés à chaque type de traitement.

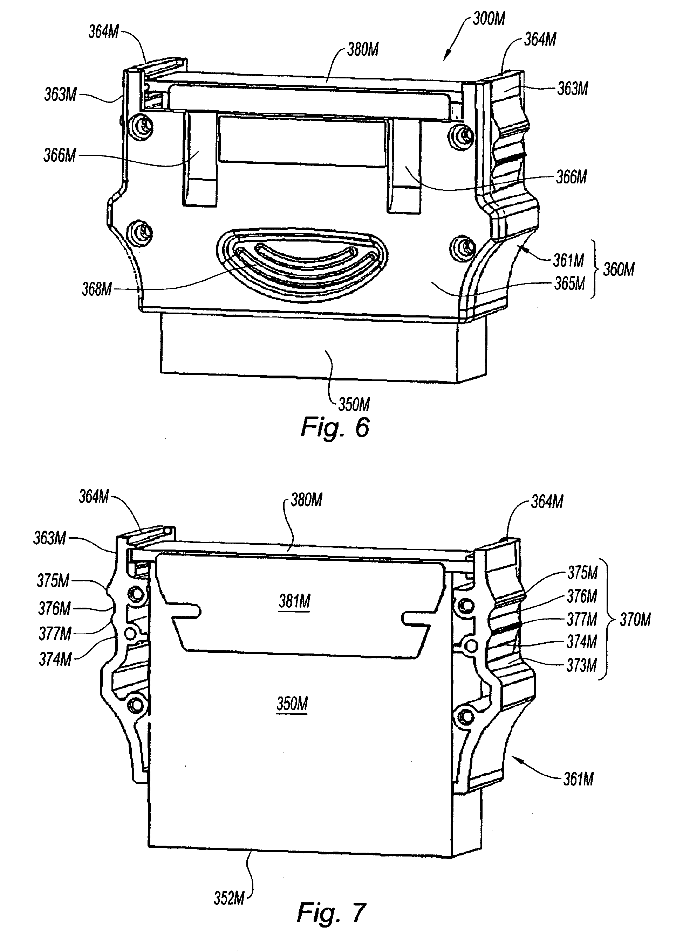

Les figures 6 à 9 montrent un autre mode de réalisation du bloc optique, facilitant sa réalisation, sa diversification et son insertion dans son logement du boîtier.

Pour la description de cette variante, on utilisera les mêmes références que celles du premier mode de réalisation, complétées par le suffixe M pour désigner les éléments identiques ou de fonction analogue à celle du premier mode de réalisation.

La description donnée ci-après se limitera aux seules différences et celle des éléments communs ne sera pas reprise pour les références identiques, complétées du suffixe M.

Selon la figure 6, le bloc optique amovible 300M se compose d'un boîtier 360M logeant un guide de lumière 350M surmonté d'une plaquette-interface 380M appliquée directement contre la face d'entrée 351 M du guide de lumière 350. Cette plaquette-interface 380M est elle-même destinée à être appliquée aussi étroitement que possible contre la face extérieure de la vitre de la fenêtre 401 du cartouche 400 de la source lumineuse.

Le boîtier 360M se compose d'une première partie 361M ayant un fond 362M, plat, le cas échéant nervuré intérieurement et bordé par deux petits côtés 363M servant de surfaces de guidage et d'enclipsage du bloc optique 300M dans le logement de la pièce à main. La première partie 361 M a ainsi une section en U, ouverte en haut et en bas. La profondeur de cette partie est adaptée à l'épaisseur du guide de lumière 350 de sorte que la deuxième partie 365M, plate, de forme adaptée au contour défini par les tranches des petits côtés 363M, forme un couvercle qui ferme le boîtier 360M en laissant dégagés le haut et le bas.

Les petits côtés 363M dépassent du grand côté et du couvercle pour former chacun une rainure 369M recevant le bord du petit côté de la plaquette-interface 380M. Cette rainure est ouverte du côté du couvercle et fermée du côté du fond. Les deux rainures parallèles 369M sont suffisamment éloignées des côtés du guide de lumière 360M pour que la plaquette-interface 380M qu'elles tiennent, déborde des deux petits côtés du guide de lumière 350M.

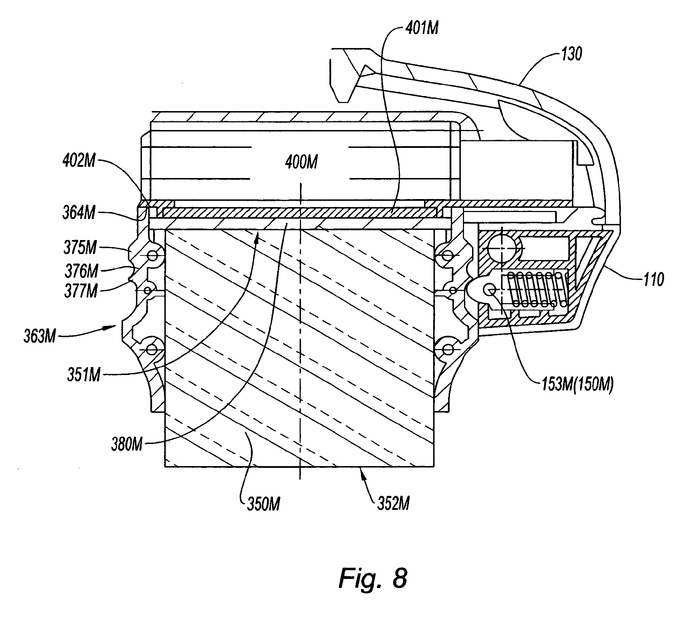

La plaquette-interface 380M se glissent librement dans les deux rainures 369M pour son remplacement par une autre plaquette-interface. La plaquette-interface 380M comme le guide de lumière 350M, sont bloqués en place dans le boîtier par le couvercle 365M. Une surface d'appui et de butée 364M définissent précisément la position du boîtier 360M et donc celle du bloc optique 300M surmonté de la plaquette-interface 380M. La sur-hauteur des deux petits côtés 363M est fixée en fonction de la place disponible autour de la fenêtre 401M de la cartouche 400 (voir figure 8).

Si le mouvement d'engagement du boîtier 360M n'est pas rigoureux et que la face supérieure de la plaquette 380M est inclinée par rapport à la fenêtre 401 M, il n'y aura pas de risque de contact ponctuel ou linéaire avec la fenêtre puisque la surface d'appui 364A la plus en avant de l'un des deux côtés 363M arrivera d'abord en appui contre le contour en retrait 402M de la fenêtre 401M et ensuite, le boîtier 360M basculera de sorte que la plaquette-interface 380M s'appliquera à plat contre la fenêtre 401M. Cela évite tout risque d'endommagement de la fenêtre 401M et/ou de la plaquette-interface 380M.

Le système optique constitué par le guide de lumière 350M et la plaquette-interface 380M permet d'obtenir de multiples caractéristiques de filtre optique d'une manière très simple. Le guide de lumière 350M est, de préférence, un élément en quartz sans caractéristiques optiques particulières de filtrage avec seulement une face d'entrée 351 M et une face de sortie 352M de lumière et des faces latérales ayant un poli de miroir pour que la fonction de guide de lumière soit aussi bonne que possible.

Le guide de lumière 350M peut avoir une forme de parallélépipède rectangle ou de pyramide tronquée suivant la répartition que l'on souhaite donner au flux lumineux en sortie du guide de lumière.

L'effet de filtre est avantageusement réalisé par la plaquette-interface 380M qui est une simple plaquette rectangulaire dont une face, de préférence celle tournée vers le guide de lumière, comporte le revêtement filtrant ou encore teintée dans la masse, suivant l'effet de filtrage plus ou moins précis. Le revêtement ou la teinte dans la masse sont fonction de la longueur d'onde voulue. Le revêtement filtrant s'obtient par un dépôt à la vapeur.

Ce procédé de dépôt métallique à la vapeur relève de la technique des couches minces. Ce ou ces revêtements peuvent être déposés directement sur la face d'entrée du guide de lumière dans le premier mode de réalisation et, de préférence dans la présente variante, sur la plaquette-interface.

La réalisation du système optique sous la forme d'un guide de lumière neutre et d'une plaquette-interface permet également d'appliquer très facilement d'autres techniques de filtrage de la lumière pour limiter celle-ci à une certaine longueur d'ondes avec une plaquette teintée dans la mase ce qui est certes possibles mais très coûteux dans le cas d'un guide de lumière.

Le revêtement filtrant appliqué sur la face de la plaquette-interface 380M tournée vers le guide de lumière 350M sera automatiquement protégé contre tout contact physique tel que le toucher par les doigts du manipulateur. Comme la plaquette 380M seule a les caractéristiques filtrantes requises, cela simplifie la fabrication des blocs optiques et leur adaptation au filtre optique particulier à réaliser. Cela se fait par le simple choix de la plaquette-interface, le guide de lumière 350M étant le même pour toutes les applications.

Selon la figure 7 qui montre le boîtier 360M sans couvercle, la grande face du guide de lumière 350M est couverte dans sa partie haute par une plaque métallique réfléchissante 381M, complétant l'effet de la surface réfléchissante (poli de miroir) de la face du guide de lumière de manière à protéger le couvercle 365M contre le rayonnement lumineux qui peut être intense et traverser le dioptre de la face du guide de lumière 350M. Cette plaquette métallique 381M est simplement tenue en place par le serrage du couvercle 365M. Une plaque identique est prévue sur l'autre grand côté du guide de lumière dans les mêmes conditions que celles décrites ci-dessus.

La figure 7 montre également le profil des deux petits côtés 363M et leurs contacts linéaires avec le guide de lumière 350M. Le guide de lumière est tenu dans son logement par serrage sur les côtés et blocage sur le dessus par la plaquette-interface 380M. Pour éviter tout mouvement de coulissement du guide de lumière, sa forme peut être légèrement trapézoïdale, le grand côté étant celui tourné vers la plaquette-interface 380M qui le bloquera.

Les petits côtés 363M de cette variante de bloc optique 300M ont un bossage dédoublé 375M-377M de hauteur réduite par rapport au bossage 372 du premier mode de réalisation. Les deux bossages forment entre eux une cuvette 376M de façon à créer un effet haptique permettant à l'opérateur qui installe le bloc optique dans le logement, de détecter le premier bossage 375M et ainsi la bonne position d'introduction du boîtier. Puis, il peut passer ce double bossage 375M (un bossage de chaque côté) de faible hauteur et offrant donc une faible résistance à l'introduction ce qui évite toute poussée excessive sur le bloc 300M qui pourrait le désaxer. Puis, après le passage en douceur dans la cuvette intermédiaire 376M et le second bossage 377M, le bloc optique est bien mis en place par la tête 153M des pistons 151 M, sur la rampe 374M ou au-delà de celle-ci dans la cuvette 373M.

Si, comme le montre la figure 8, la géométrie des petits côtés 363M et celle de la position des pistons 151M est définie pour que la tête 153M des pistons 151 M reste sur la rampe 374M, on crée ainsi une composante de poussée longitudinale s'exerçant sur le bloc optique 300M pour l'appliquer dans son logement contre la fenêtre 401M du cartouche 400M.

La figure 8 montre également la venue en appui des surfaces d'appui 374M des petits côtés 373M contre le fond du contour 402M de la fenêtre 401 M ainsi que l'application étroite de la plaquette interface 380M contre la fenêtre 401M et contre la face d'entrée 351M du guide de lumière 350M.

**NOMENCLATURE**

| | |
|---|---|
| 100 | pièce à main |
| 110 | coque |
| 110a, b | parties de la coque |
| 119 | poignée |
| 120 | tête |
| 130 | capot |
| 140 | logement du bloc optique |
| 141 | nervures |
| 142 | guide |
| 143 | surface de guidage |
| 150, 151 M | élément d'enclipsage |
| 151 | piston |
| 152 | logement transversal |
| 153, 153M | tête de piston |
| 154 | ressort |
| | |
| 200 | faisceau de câbles et de tuyaux |
| | |
| 300 | bloc optique amovible |
| 350, 350M | guide de lumière |
| 350A | forme parallélépipédique |
| 350B | forme prismatique |
| 351, 351M | face d'entrée |
| 352, 352M | face de sortie |
| 360, 360M | boîtier du bloc optique |
| 361, 361 M | première partie |
| 362 | fond rectangulaire |
| 362A, B | grandes faces extérieures |
| 363, 363M | petit côté |
| 364M | surface d'appui |
| 365, 365M | seconde partie |
| 365A | grande face extérieure |
| 365B | face intérieure du couvercle 365 |
| 366, 366M | rainures de guidage longitudinal |
| 368, 368M | cavité de préhension |
| 369M | rainure |
| 370 | surface |
| 371,372 | bossages |
| 373, 373M | cuvette |
| 374, 374M | rampe |
| 375M | bossage |
| 376M | cuvette |
| 377M | bossage |
| 380M | plaquette-interface |
| 381M | plaque métallique |
| | |
| 400M | cartouche |
| 401M | fenêtre |
| 402M | contour en retrait |

## Revendications

1. Pièce à main d'appareil de phototraitement de la peau, pièce formée d'une coque (100) avec une poignée (110) reliée à un faisceau (200) de câbles d'alimentation électrique et de tubes de fluide caloporteur et une tête, recevant une cartouche munie de la source lumineuse commandée et d'un guide de lumière (350) alimenté par la source lumineuse et dont la surface de sortie (352) constitue la surface d'émission du signal lumineux vers la surface de la peau à traiter,
**caractérisée en ce qu'**
elle comprend
- un logement (140) débouchant, réalisé dans la coque (110), arrivant sous la surface d'émission de la cartouche et muni de moyens de guidage (141, 142, 143) et d'organes d'enclipsage (150),
- un bloc optique (300) interchangeable portant le guide de lumière (350) et engagé de façon amovible dans le logement (140), ce bloc optique ayant un boîtier (360) recevant le guide de lumière (350) et muni de surfaces de guidage et d'enclipsage (366, 371, 372, 373, 374) pour coopérer avec les moyens de guidage (141, 142, 143) et les organes d'enclipsage escamotable (150) du logement (140).

2. Pièce à main selon la revendication 1,
**caractérisée en ce que**
les organes d'enclipsage (150) sont constitués par des butées à ressort en forme de piston (151) dont la tête (153) est en saillie de la surface de guidage (143) du logement (140) et le boîtier (360) du bloc optique comporte des surfaces de guidage (370) munies d'une cavité (373) formant une rampe (374) pour recevoir la tête du piston (151) de l'organe d'enclipsage (150) associé.

3. Pièce à main selon la revendication 1,
**caractérisée en ce que**
le boîtier (360) est constitué par un élément de forme parallélépipédique, de section globalement rectangulaire, composé d'une première partie (361) avec un fond (362) et deux côtés (363) ainsi qu'une seconde partie (365) en forme de couvercle pour constituer entre elles une cavité débouchant des deux côtés pour recevoir le guide de lumière (350), les deux parties étant fixées l'une à l'autre pour bloquer le guide de lumière et les côtés (363) formant les surfaces de guidage et d'enclipsage (370).

4. Pièce à main selon la revendication 3,
**caractérisée en ce que**
le boîtier (360) comporte des rainures (366) réalisées dans les grands côtés pour coopérer avec des nervures de guidage (142) homologues du logement (140) de la coque (110).

5. Pièce à main selon la revendication 1,
**caractérisée en ce que**
dans sa surface qui dépasse du logement (140) de la coque lorsque le bloc optique (300) y est en place, le boîtier (360) comporte sur deux grandes faces (362A, 365A), des cavités de préhension (368).

6. Pièce à main selon la revendication 1,
**caractérisée en ce que**
le bloc optique est muni d'un système de refroidissement du guide de lumière utilisant par exemple l'effet Pelletier.

7. Pièce à main selon la revendication 1,
**caractérisée en ce qu'**
elle comprend un bloc optique aveugle pour se placer dans le logement de la pièce à main, fermer celui-ci et éviter la pénétration de poussière.

8. Pièce à main selon la revendication 1,
**caractérisée en ce que**
le bloc optique (300M) comporte une plaquette-interface (380M) appliquée contre la face d'entrée (351M) du guide de lumière (350M) et ayant des caractéristiques optiques pour la transmission de la lumière entre la source lumineuse de la cartouche (400M) et le guide de lumière (350M).

9. Pièce à main selon la revendication 8,
**caractérisée en ce que**
la plaquette-interface (380M) a une face traitée, un filtre optique ou un traitement dans la masse pour former un filtre optique.

10. Pièce à main selon la revendication 8,
**caractérisée en ce que**
le bloc optique (300M) a un boîtier dont la première partie comporte un logement recevant le guide optique (350M) et au-dessus du logement, deux rainures (379M) dans les petits côtés (363M) recevant la plaquette-interface (380M).

11. Pièce à main selon la revendication 2,
**caractérisée en ce que**
le boîtier (360M) du bloc optique comporte des surfaces de guidage (370M) munies d'une cavité (373M) formant une rampe (374M) et précédées dans le sens de l'engagement du bloc optique (300M) dans son logement de la coque (110) par deux bossages (375M, 377M) dont le premier (375M) assure le positionnement du bloc optique (300M) dans l'entrée du logement et le second bossage (377M) délimite la cavité (373M) recevant la tête (153M) du piston (151M) de chacun des organes d'enclipsage (150M).

12. Pièce à main selon la revendication 1,
**caractérisée en ce que**
le bloc optique (300M) comporte sur les deux faces du guide de lumière (350M), un réflecteur en forme de plaque métallique (381M) appliquée contre chaque grande face du guide de lumière (350M) pour protéger le boîtier (360M) contre la surchauffe.

## Patentansprüche

1. Handstück für Geräte zur Lichtbehandlung der Haut, wobei das Handstück aus einer Schale (100) mit einem Griff (110) gebildet ist, der mit einem Strang (200) von Stromversorgungskabeln und Leitungen für ein Wärmeträgermedium verbunden ist, sowie aus einem Kopf, der eine Kartusche aufnimmt, die mit der gesteuerten Lichtquelle und einem Lichtleiter (350) versehen ist, der von der Lichtquelle gespeist wird und dessen Austrittsfläche (352) die Fläche zum Aussenden des Lichtsignals zur Oberfläche der zu behandelnden Haut bildet,
**dadurch gekennzeichnet, dass**
es enthält
- eine durchgehende Aufnahmeausnehmung (140), die in die Schale (110) eingebracht ist, unter die Sendefläche der Kartusche reicht und mit Führungsmitteln (141, 142, 143) und Einschnappgliedern (150) versehen ist,
- einen auswechselbaren optischen Block (300), der den Lichtleiter (350) trägt und abnehmbar in die Aufnahmeausnehmung (140) eingreift, wobei dieser optische Block ein Gehäuse (360) aufweist, das den Lichtleiter (350) aufnimmt und mit Führungs- und Einschnappflächen (366, 371, 372, 373, 374) versehen ist, um mit den Führungsmitteln (141, 142, 143) und den einklappbaren Einschnappgliedern (150) der Aufnahmeausnehmung (140) zusammenzuwirken.

2. Handstück nach Anspruch 1,
**dadurch gekennzeichnet, dass**
die Einschnappglieder (150) aus Federanschlägen in Form von Kolben (151) bestehen, deren Kopf (153) von der Führungsfläche (143) der Aufnahmeausnehmung (140) vorspringt, und dass das Gehäuse (360) des optischen Blocks Führungsflächen (370) aufweist, die mit einem Hohlraum (373) versehen sind, der eine Rampe (374) zum Aufnehmen von dem Kopf des Kolbens (151) des zugeordneten Einschnappglieds (150) bildet.

3. Handstück nach Anspruch 1,
**dadurch gekennzeichnet, dass**
das Gehäuse (360) aus einem quaderförmigen Teil mit rechteckförmigem Querschnitt besteht, das aus einem ersten Abschnitt (361) mit einem Boden (362) und zwei Seiten (363) sowie aus einem zweiten Abschnitt (365) in Form eines Deckels besteht, um dazwischen einen Hohlraum zu bilden, der auf beiden Seiten ausmündet, um den Lichtleiter (350) aufzunehmen, wobei die beiden Abschnitte aneinander befestigt sind, um den Lichtleiter und die Seiten (363), welche die Führungs- und Einschnappflächen (370) bilden, zu sichern.

4. Handstück nach Anspruch 3,
**dadurch gekennzeichnet, dass**
das Gehäuse (360) Rillen (366) aufweist, die in den Längsseiten ausgeführt sind, um mit entsprechenden Führungsrippen (142) der Aufnahmeausnehmung (140) der Schale (110) zusammenzuwirken.

5. Handstück nach Anspruch 1,
**dadurch gekennzeichnet, dass**
in dessen Fläche, die von der Aufnahmeausnehmung (140) der Schale dann vorsteht, wenn der optische Block (300) darin eingesetzt ist, das Gehäuse (360) auf zwei größeren Flächen (362A, 365A) Greifmulden (368) aufweist.

6. Handstück nach Anspruch 1,
**dadurch gekennzeichnet, dass**
der optische Block mit einer Einrichtung zum Abkühlen des Lichtleiters beispielsweise unter Nutzung des Pelletier-Effekts versehen ist.

7. Handstück nach Anspruch 1,
**dadurch gekennzeichnet, dass**
es einen blinden optischen Block aufweist, um diesen in die Aufnahmeausnehmung des Handstücks einzusetzen, diese zu verschließen und das Eindringen von Staub zu vermeiden.

8. Handstück nach Anspruch 1,
**dadurch gekennzeichnet, dass**
der optische Block (300M) eine Schnittstellenplatte (380M) aufweist, die an die Eintrittsfläche (351M) des Lichtleiters (350M) angelegt ist und optische Eigenschaften zum Übertragen von Licht zwischen der Lichtquelle der Kartusche (400M) und dem Lichtleiter (350M) hat.

9. Handstück nach Anspruch 8,
**dadurch gekennzeichnet, dass**
die Schnittstellenplatte (380M) eine behandelte Fläche, ein optisches Filter oder eine Massebearbeitung zum Bilden eines optischen Filters aufweist.

10. Handstück nach Anspruch 8,
**dadurch gekennzeichnet, dass**
der optische Block (300M) ein Gehäuse hat, dessen erster Abschnitt eine Aufnahmeausnehmung aufweist, die den optischen Leiter (350M) aufnimmt, und oberhalb der Aufnahmeausnehmung zwei Rillen (379M) in den Schmalseiten (363M) enthält, welche die Schnittstellenplatte (380M) aufnehmen.

11. Handstück nach Anspruch 2,
**dadurch gekennzeichnet, dass**
das Gehäuse (360M) des optischen Blocks Führungsflächen (370M) aufweist, die mit einem Hohlraum (373M) versehen sind, der eine Rampe (374M) bildet, und denen in Eingriffsrichtung des optischen Blocks (300M) in seine Aufnahmeausnehmung der Schale (110) zwei Erhebungen (373M, 377M) vorangehen, von denen die erste (375M) die Positionierung des optischen Blocks (300M) im Eintritt der Aufnahmeausnehmung sicherstellt und die zweite Erhebung (377M) den Hohlraum (373M) eingrenzt, der den Kopf (153M) des Kolbens (151M) eines jeden der Einschnappglieder (150M) aufnimmt.

12. Handstück nach Anspruch 1,
**dadurch gekennzeichnet, dass**
der optische Block (300M) an den beiden Seiten des Lichtleiters (350M) einen Reflektor in Form von einer Metallplatte (381M) aufweist, die an jeder großen Fläche des Lichtleiters (350M) anliegt, um das Gehäuse (360M) vor Überhitzung zu schützen.

## Claims

1. A handpiece for a skin phototreatment apparatus, which piece is formed by a shell (100) with a handle (110) connected to a bundle (200) of electrical power supply cables and heat transfer fluid tubes and a head, receiving a cartridge provided with a controlled light source and with a light guide (350) fed by the light source and the output surface (352) of which forms the surface of emission of the light signal towards the surface of the skin to be treated, **characterized in that**
it comprises
- a housing (140) with an opening, which housing being formed in the shell (110) and extending to beneath the emission surface of the cartridge, and provided with guiding means (141, 142, 143) and clip-fit components (150),
- an interchangeable optical unit (300) which carries the light guide (350) and is removably slotted into the housing (140), this optical unit having a casing (360) which receives the light guide (350) and is provided with guiding and clip-fit surfaces (366, 371, 372, 373, 374) for cooperating with the guiding means (141, 142, 143) and the retractable clip-fit components (150) of the housing (140).

2. A handpiece according to Claim 1,
**characterized in that**
the clip-fit components (150) are formed by spring-loaded stops in the form of a piston (151), the head (153) of which protrudes from the guiding surface (143) of the housing (140), and the casing (360) of the optical unit includes guiding surfaces (370) provided with a recess (373) forming a ramp (374) for receiving the head of the piston (151) of the associated clip-fit component (150).

3. A handpiece according to Claim 1,
**characterized in that**
the casing (360) is formed by a parallelepiped-shaped element, of rectangular overall cross-section, which is composed of a first part (361) having a base (362) and two sides (363) and of a second part (365) in the form of a cover, which parts form between them a cavity opening out on two sides for receiving the light guide (350), the two parts being fixed to one another in order to secure the light guide and the sides (363) forming the guiding and clip-fit surfaces (370).

4. A handpiece according to Claim 3,
**characterized in that**
the casing (360) includes grooves (366) which are formed in the large sides to cooperate with homologous guiding ribs (142) of the housing (140) of the shell (110).

5. A handpiece according to Claim 1,
**characterized in that**
in its surface which protrudes from the housing (140) of the shell when the optical unit (300) is in place therein, the casing (360) includes grip recesses (368) on two large faces (362A, 365A).

6. A handpiece according to Claim 1,
**characterized in that**
the optical unit is provided with a system for cooling the light guide using for example the Peltier effect.

7. A handpiece according to Claim 1,
**characterized in that**
it comprises a blind optical unit to be placed in the housing of the handpiece, to close the latter and prevent the penetration of dust.

8. A handpiece according to Claim 1,
**characterized in that**
the optical unit (300M) comprises an interface-plate (380M) applied on the input face (351M) of the light guide (350M) and having optical characteristics for the transmission of light between the light source of the cartridge (400M) and the light guide (350M).

9. A handpiece according to Claim 8,
**characterized in that**
the interface-plate (380M) has a treated face, an optical filter or an in-the-mass treatment to form an optical filter.

10. A handpiece according to Claim 8,
**characterized in that**
the optical unit (300M) has a casing, the first part of which includes a housing which receives the optical guide (350M) and, above the housing, two grooves (379M) in the small sides (363M) which receive the interface-plate (380M).

11. A handpiece according to Claim 2,
**characterized in that**
the casing (360M) of the optical unit includes guiding surfaces (370M) which are provided with a recess (373M) forming a ramp (374M) and are preceded in the direction of insertion of the optical unit (300M) into its housing in the shell (110) by two projections (375M, 377M), the first (375M) of which serves to position the optical unit (300M) in the entrance of the housing, and the second projection (377M) delimits the recess (373M) receiving the head (153M) of the piston (151M) of each of the clip-fit components (150M).

12. A handpiece according to Claim 1,
**characterized in that**
the optical unit (300M) includes, on the two faces of the light guide (350M), a reflector in the form of a metal plate (381M) applied on each large face of the light guide (350M) in order to protect the casing (360M) from overheating.
